# EUROPEAN PATENT APPLICATION

(11) **EP 4 703 467 A1**
(43) Date of publication of application: **04.03.2026**
(21) Application number: 24797039.5
(22) Date of filing: 24.04.2024
(51) Int. Cl.: C12N 5/10, C07K 14/705, C12N 15/12, C12Q 1/02

(54) **METHOD FOR PRODUCING CELLS FOR MEASURING ACTIVITY OF SENSOR PROTEIN**

(30) Priority: 28.04.2023 JP 2023075288
(71) Applicant: Sumitomo Chemical Company, Limited, Tokyo 103-6020 (JP)
(72) Inventor: SASAOKA, Norio, Osaka-shi, Osaka 554-8558 (JP); SEKIGUCHI, Michiru, Osaka-shi, Osaka 554-8558 (JP); TAKAHASHI, Yasuhiko, Osaka-shi, Osaka 554-8558 (JP)
(74) Representative: Vossius & Partner Patentanwälte Rechtsanwälte mbB
(86) International application number: PCT/JP2024/016006
(87) International publication number: WO 2024/225293

(57) **Abstract**

Provided is a method for producing cells for measuring the activity of a sensor protein that can be used as a chemical sensor through culture. The method for producing cells for measuring the activity of a sensor protein includes culturing cells containing a polynucleotide containing a coding sequence for the sensor protein, and collecting the cells when the ratio of the activity value of the sensor protein at a concentration of 1 uM of a response substance to the activity value of the sensor protein at a concentration of 0 µM of the response substance (specific activity) is 1.2 or greater.

## Description

### Technical Field

The present invention relates to a method for producing cells for measuring the activity of a sensor protein and the like.

### Background Art

Groups of odorous substances associated with specific human diseases and mental states have been identified. Owing to their high value as diagnostic markers, there has been growing interest in developing various odor sensors that target these substances. Biological odorant receptors exhibit excellent characteristics, including diversity, sensitivity, and selectivity, which are not found in conventional odor sensor elements such as semiconductors; thus, these odorant receptors offer a promising foundation for the development of novel odor sensors using them as sensor elements.

PTL 1 discloses the use of cells expressing modified odorant receptors or lipid bilayer membranes incorporating modified odorant receptors as odor sensors.

### Citation List

### Patent Literature

PTL 1: WO2022/024902A

### Summary of Invention

### Technical Problem

Odor sensors that rely on the process of artificially preparing lipid bilayer membranes equipped with sensor proteins (e.g., odorant receptors) are often inefficient to produce, and there has been demand for further improvement in the production efficiency of odor sensors. The present inventors then focused on using cells that express sensor proteins.

From the perspective of convenience in use, the cells used as a chemical sensor such as an odor sensor are desirably those prepared in advance and retained in containers, which can be used when needed, rather than cells that are cultured and prepared each time for use. In this case, from the perspective of business economics, it is necessary to culture cells functioning as chemical sensors beforehand.

An object of the present disclosure is to provide a method for producing cells for measuring the activity of sensor proteins that can be used as chemical sensors through culture.

### Solution to Problem

In the course of their research, the present inventors found that while the activity of sensor proteins may be almost completely lost due to culture in some cases, it is possible to maintain the activity. Based on this finding, they conducted further research and discovered that the problem can be solved by a method for producing cells for measuring the activity of a sensor protein, comprising culturing cells containing a polynucleotide containing a coding sequence for the sensor protein, and collecting the cells when the ratio of the activity value of the sensor protein at a concentration of 1 µM of a response substance to the activity value of the sensor protein at a concentration of 0 µM of the response substance (specific activity) is 1.2 or greater. Based on this finding, the inventors further advanced their research and completed the invention of the present disclosure. Specifically, the present disclosure encompasses the following embodiments.

### Item 1.

A method for producing a cell for measuring activity of a sensor protein, comprising
culturing a cell containing a polynucleotide containing a coding sequence for the sensor protein, and
collecting the cell when the ratio of the activity value of the sensor protein at a concentration of 1 µM of a response substance to the activity value of the sensor protein at a concentration of 0 µM of the response substance (specific activity) is 1.2 or greater.

### Item 2.

The method according to Item 1, wherein the cell concentration at the time of the cell collection is less than 2.9 × 10⁶ cells/mL.

### Item 3.

The method according to Item 2, wherein the cell concentration at the time of the cell collection is 2.2 × 10⁶ cells/mL or less.

### Item 4.

The method according to Item 3, wherein the cell concentration at the time of the cell collection is 2.0 × 10⁵ cells/mL or more.

### Item 5.

The method according to Item 4, wherein the cell concentration at the time of the cell collection is 8.7 × 10⁵ to 1.75 × 10⁶ cells/mL.

### Item 6.

The method according to Item 1, wherein the culture period from a passage immediately before the cell collection to the cell collection is 6 to 12 days.

### Item 7.

The method according to Item 1, comprising seeding the insect cell so as to achieve a cell concentration of 0.8 × 10⁵ to 2.0 × 10⁵ cells/mL at the time of a passage immediately before the cell collection.

### Item 8.

The method according to Item 7, wherein the cell concentration is 0.8 × 10⁵ to 1.5 × 10⁵ cells/mL.

### Item 9.

The method according to any one of Items 1 to 8, wherein the cell is an insect cell.

### Item 10.

The method according to any one of Items 1 to 8, wherein the sensor protein is an odorant receptor protein.

### Item 11.

A cell comprising a polynucleotide containing a coding sequence for a sensor protein,
wherein the cell is obtained through a culture step, and
wherein the ratio of the activity value of the sensor protein at a concentration of 1 µM of a response substance to the activity value of the sensor protein at a concentration of 0 µM of the response substance (specific activity) is 1.2 or greater.

### Advantageous Effects of Invention

The present disclosure provides a method for producing cells for measuring the activity of a sensor protein that can be used as a chemical sensor through culture.

### Description of Embodiments

In the present specification, the terms "comprising," "containing," and "including" include the concepts of comprising, containing, consisting essentially of, and consisting of.

In an embodiment, the present disclosure relates to a method for producing cells for measuring the activity of a sensor protein ("the production method of the present disclosure" in the present specification), comprising culturing cells containing a polynucleotide containing a coding sequence for the sensor protein, and collecting the cells when the ratio of the activity value of the sensor protein at a concentration of 1 µM of a response substance to the activity value of the sensor protein at a concentration of 0 µM of the response substance (specific activity) is 1.2 or greater. The following provides an explanation of this method.

The polynucleotide may be endogenous (a polynucleotide consisting of a base sequence within the genome of that cell) or exogenous. The "exogenous polynucleotide" is not particularly limited as long as it refers to a polynucleotide containing a base sequence that is not derived from the genomic DNA (in particular, chromosomal genomic DNA) of the cell.

The cell used in the production method of the present disclosure preferably contains an exogenous polynucleotide comprising a coding sequence for a sensor protein. This enables the expression of any sensor protein and enables increased expression levels of a target sensor protein, thereby enhancing the detection sensitivity for a target chemical substance. The production method of the present disclosure is particularly suitable for such cells.

In the present specification, the polynucleotide includes not only typical polynucleotides such as DNA and RNA inherent in organisms, but also polynucleotides with known chemical modifications, artificial polynucleotides, and like polynucleotides, as listed below. To prevent degradation due to hydrolases such as nucleases, the phosphate residue of each nucleotide can be substituted with a chemically modified phosphate residue, such as phosphorothioate (PS), methylphosphonate, or phosphorodithionate. The hydroxyl group at position 2 of the ribose of each ribonucleotide may also be substituted with -OR (R indicates, for example, CH₃(2'-O-Me), CH₂CH₂OCH₃(2'-O-MOE), CH₂CH₂NHC(NH)NH₂, CH₂CONHCH₃, or CH₂CH₂CN). Additionally, the nucleobase moiety (pyrimidine, purine) may be chemically modified, by, for example, introduction of a methyl group or a cationic functional group into position 5 of the pyrimidine base, or substitution of the carbonyl group at position 2 with thiocarbonyl. Additionally, the polynucleotide of the present invention also includes, but is not limited to, those formed by modifying the phosphate moiety or the hydroxyl moiety, for example, with biotin, an amino group, a lower alkyl amine group, or an acetyl group. The polynucleotide for use can also be, for example, BNA (LNA), which is prepared by crosslinking the 2' oxygen and the 4' carbon in the ribose moiety of a nucleotide to fix the ribose moiety in N-conformation.

The sensor protein may be selected from proteins capable of detecting the presence of a chemical substance, such as a receptor protein that uses a chemical substance as a ligand. The sensor protein is particularly preferably an odorant receptor protein.

The insect odorant receptor protein is a membrane protein with a seven-transmembrane structure and functions as an odor sensor in the organisms. The odorant receptor protein is composed of the following components that are sequentially linked from the amino terminus ("N-terminus" below) to the carboxyl terminus ("C-terminus" below): N-terminal region (NT), first transmembrane domain (TM1), first extracellular loop (EC1), second transmembrane domain (TM2), first intracellular loop (IC1), third transmembrane domain (TM3), second extracellular loop (EC2), fourth transmembrane domain (TM4), second intracellular loop (IC2), fifth transmembrane domain (TM5), third extracellular loop (EC3), sixth transmembrane domain (TM6), third intracellular loop (IC3), seventh transmembrane domain (TM7), and C-terminal region (CT). In the present disclosure, each region is determined by structural prediction using TMpred (K. Hofmann, W. Stoffel, TMbase - a database of membrane spanning proteins segments, Biol. Chem. Hoppe-Seyler, 374 (1993), p. 166, https://embnet.vital-it.ch/software/TMPRED_form.html) (with default conditions).

The insect of origin for the insect odorant receptor protein is preferably an insect in the order *Diptera,* such as the family *Culicidae* and the family *Drosophilidae;* an insect in the order *Lepidoptera,* such as the family *Bombycidae;* an insect in the order *Hymenoptera,* such as the family *Apidae;* an insect in the order *Orthoptera,* such as the family *Acrididae;* and an insect in the order *Hemiptera,* such as the family *Cimicidae,* and more preferably an insect in the order *Diptera,* such as the family *Culicidae* and the family *Drosophilidae;* an insect in the order *Orthoptera,* such as the family *Acrididae;* and an insect in the order *Hemiptera,* such as the family *Cimicidae.* Examples of insects in the family *Culicidae* include *Anopheles gambiae, Aedes aegypti,* and *Culex quinquefasciatus.* Examples of insects in the family *Drosophilidae* include *Drosophila melanogaster, Drosophila pseudoobscura,* and *Drosophila virilis.* Examples of insects in the family *Bombycidae* include *Bombyx mori, Bombyx mandarina,* and *Trilocha varians.* Examples of insects in the family *Apidae* include *Apis mellifera, Apis florea, Apis dorsata,* and *Bombus terrestris.* Examples of insects in the family *Acrididae* include *Locusta migratoria.* Examples of insects in the family *Cimicidae* include *Cimex lectularius.*

Specifically, examples of wild-type insect odorant receptor proteins include the following: AaOR1, AaOR2, AaOR4, AaOR5, AaOR6, AaOR8, AaOR9, AaOR10a, AaOR15, AaOR22, AaOR24, AaOR25, AaOR26, AaOR27, AaOR28, AaOR30, AaOR34, AaOR36, AaOR38, AaOR41a, AaOR41b, AaOR42, AaOR43, AaOR44, AaOR47, AaOR49, AaOR50, AaOR52, AaOR54, AaOR58, AaOR59, AaOR60, AaOR61, AaOR64, AaOR65, AaOR66, AaOR67a, AaOR69a, AaOR70, AaOR71, AaOR72a, AaOR73, AaOR74, AaOR75, AaOR77, AaOR78, AaOR79, AaOR81, AaOR83b, AaOR84, AaOR85, AaOR86, AaOR87, AaOR91, AaOR95, AaOR97, AaOR96, AaOR99, AaOR100, AaOR102, AaOR103, AaOR104a, AaOR105, AaOR107, AaOR108, AaOR109, AaOR110, AaOR112, AaOR114, AaOR116, AaOR117, AaOR118, AaOR122, AaOR125, AaOR128, AgOR1, AgOR2, AgOR3, AgOR4, AgOR5, AgOR6, AgOR8, AgOR9, AgOR10, AgOR11a, AgOR12a, AgOR12b, AgOR13, AgOR14, AgOR15, AgOR16a, AgOR17, AgOR18, AgOR20, AgOR21, AgOR23, AgOR25, AgOR26, AgOR27, AgOR28, AgOR30, AgOR34, AgOR36, AgOR37, AgOR38, AgOR39a, AgOR40, AgOR42, AgOR44, AgOR45, AgOR46, AgOR47, AgOR49, AgOR50, AgOR54, AgOR56a, AgOR57, AgOR60, AgOR61, AgOR62, AgOR63, AgOR64, AgOR65, AgOR69, AgOR70, AgOR71, AgOR72, AgOR74, AgOR75, AgOR76a, AmOR1, AmOR3, AmOR9, AmOR10, AmOR13, AmOR41, AmOR51, AmOR52, AmOR55, AmOR71, AmOR73, AmOR78, AmOR85, AmOR89, AmOR90, AmOR114, AmOR115, AmOR118, AmOR120, AmOR121, AmOR161, BmOR1, BmOR2, BmOR3, BmOR4, BmOR5, BmOR8, BmOR9, BmOR10, BmOR13, BmOR17, BmOR18, BmOR23, BmOR24, BmOR25, BmOR35, BmOR36, BmOR42, BmOR45, BmOR49, BmOR51, BmOR52, BmOR55, BmOR56, BmOR61, DmOR1a, DmOR9a, DmOR19a, DmOR22a, DmOR22b, DmOR22c, DmOR24a, DmOR30a, DmOR33a, DmOR33b, DmOR33c, DmOR35a, DmOR42b, DmOR43a, DmOR45a, DmOR45b, DmOR47a, DmOR49b, DmOR59b, DmOR65b, DmOR65c, DmOR67b, DmOR67c, DmOR69a, DmOR71a, DmOR74a, DmOR82a, DmOR83a, DmOR83c, DmOR85a, DmOR85c, DmOR85e, DmOR85f, DmOR88a, DmOR92a, DmOR94a, DmOR94b, and DmOR98b.

In the present specification, OR denotes "odorant receptor." Dm indicates derivation from *Drosophila melanogaster,* Bm indicates derivation from *Bombyx mori,* Ag indicates derivation from *Anopheles gambiae,* and Aa indicates derivation from *Aedes aegypti.* The amino acid sequences of various odorant receptor proteins, including these, and their coding sequences are either known or can be readily identified through sequence identity searches based on known sequences.

The sensor protein may contain one or more amino acid mutations in its wild-type amino acid sequence, provided that the chemical response activity is not significantly decreased. The phrase "not significantly decreased" means, for example, that the chemical response activity of a sensor protein containing amino acid mutations is, for example, at least 50%, preferably at least 60%, more preferably at least 70%, even more preferably at least 80%, and yet more preferably at least 90% of the chemical response activity of the wild-type sensor protein, taken as 100%.

The amino acid mutation is, for example, substitution, insertion, addition, or deletion of an amino acid, preferably substitution, and particularly preferably conservative substitution.

In the present specification, "conservative substitution" refers to the substitution of an amino acid residue with another amino acid residue having a similar side chain. For example, the substitution between amino acid residues having a basic side chain such as lysine, arginine, or histidine is considered to be a conservative substitution. The following substitutions between other amino acid residues are also considered to be a conservative substitution: the substitution between amino acid residues having an acidic side chain such as aspartic acid and glutamic acid; the substitution between amino acid residues having an uncharged polar side chain such as glycine, asparagine, glutamine, serine, threonine, tyrosine, or cysteine; the substitution between amino acid residues having a nonpolar side chain such as alanine, valine, leucine, isoleucine, proline, phenylalanine, methionine, or tryptophan; the substitution between amino acid residues having a β-branched side chain such as threonine, valine, or isoleucine; and the substitution between amino acid residues having an aromatic side chain such as tyrosine, phenylalanine, tryptophan, or histidine.

The sensor protein may contain its wild-type amino acid sequence or an amino acid sequence having, for example, at least 70%, preferably at least 80%, more preferably at least 90%, even more preferably at least 95%, still more preferably at least 98%, and particularly preferably at least 99% identity to the wild-type amino acid sequence.

In the present specification, the "identity" of amino acid sequences refers to the degree to which two or more contrastable amino acid sequences match each other. Thus, the higher the degree of match between two amino acid sequences, the higher the identity or similarity of those sequences. The level of amino acid sequence identity is determined, for example, by using FASTA, which is a tool for sequence analysis, with default parameters. Alternatively, the level of amino acid sequence identity can be determined by using the BLAST algorithm by Karlin and Altschul (Karlin S., Altschul S. F. Methods for assessing the statistical significance of molecular sequence features by using general scorings schemes, Proc. Natl. Acad. Sci. USA. 87: 2264-2268 (1990); Karlin S., Altschul S. F. Applications and statistics for multiple high-scoring segments in molecular sequences, Proc Natl Acad Sci USA. 90: 5873-7 (1993)). A program called "BLASTX," based on this BLAST algorithm, has been developed. The specific techniques of these analysis methods are known and can be found on the website of the National Center of Biotechnology Information (NCBI) (http://www.ncbi.nlm.nih.gov/).

The sensor protein may contain other amino acid sequences, such as protein tags, fluorescent proteins, luminescent proteins, signal sequences, or other proteins or peptides, attached thereto, provided that the chemical response activity is not significantly impaired. Examples of protein tags include biotin, His tag, FLAG tag, Halo tag, MBP tag, HA tag, Myc tag, V5 tag, and PA tag.

In the present specification, "chemical response activity" refers to the property of a sensor protein to recognize a chemical substance and initiate signal transduction activity (e.g., ion channel activity) either alone or in conjunction with other proteins. In the case of odorant receptors, "chemical response activity" refers to the property of an odorant receptor to recognize a chemical substance and form an odorant receptor complex, together with an olfactory receptor co-receptor, thereby exhibiting ion channel activity upon activation. The chemical response activity of a sensor protein can be measured by using the signal transduction activity of the sensor protein in contact with a chemical substance as an indicator (e.g., by quantifying and evaluating the amount of signal molecules). In the case of odorant receptors, their chemical response activity can be measured using the ion channel activity of an odorant receptor complex formed of an odorant receptor in contact with a chemical substance and an olfactory receptor co-receptor as an indicator. For example, a chemical substance is brought into contact with cells that express a protein that becomes fluorescent or luminescent in response to the influx of ions (e.g. calcium ions) into the cells when (a) an odorant receptor, (b) an olfactory receptor co-receptor, and (c) an odorant receptor complex respond, and then the amount of luminescence of the cells is measured. The greater the measured amount of luminescence, the higher the response activity of the odorant receptor to the chemical substance is determined to be. Specifically, the chemical response activity can be measured according to the method described in PTL 1.

The coding sequence for the sensor protein is not particularly limited as long as it is a base sequence encoding the sensor protein. In an embodiment, the polynucleotide contains an expression cassette for a sensor protein. The expression cassette is not particularly limited as long as it is a polynucleotide capable of expressing the sensor protein within a cell. A typical example of expression cassettes for a sensor protein is a polynucleotide containing a promoter and the coding sequence for the sensor protein placed under control of the promoter.

The promoter is not particularly limited and can be selected as appropriate. The promoter for use can be selected from various types of pol II promoters, for example. Examples of pol II promoters include, but are not particularly limited to, CMV promoter, EF1 promoter, SV40 promoter, MSCV promoter, and promoters derived from insect genes.

When the sensor protein is an insect odorant receptor, it is preferred that the polynucleotide contain a coding sequence for an insect olfactory receptor co-receptor. The insect olfactory receptor co-receptor is a membrane protein with a seven-transmembrane structure as with the odorant receptor and functions by forming a hetero-complex with an odorant receptor. The odorant receptor complex, which is a hetero-complex composed of an odorant receptor and an olfactory receptor co-receptor, has ion channel activity that is activated by odorous substances. When activated, the odorant receptor complex allows the influx of cations such as sodium ions (Na⁺) and calcium ions (Ca²⁺) into cells.

It is preferred that the polynucleotide include a coding sequence for a protein that becomes fluorescent or luminescent in response to the influx of ions (e.g., calcium ions) into the cell when the sensor protein (in particular, the odorant receptor protein) responds. Examples of such proteins include aequorin, Yellow Cameleon (YC), and GCaMP. Alternatively, the cell of the present disclosure preferably contains an ion-dependent fluorescent dye, such as calcium ion-dependent fluorescent dyes (e.g., Fura-2, Fluo-3, and Fluo-4).

The polynucleotide preferably contains a coding sequence for a drug-resistant gene to allow for drug screening of insect cells. For the drug-resistant gene, a gene resistant to a drug that can be used for drug screening of insect cells may be selected. Examples of drug-resistant genes include chloramphenicol resistance gene, tetracycline resistance gene, neomycin resistance gene, erythromycin resistance gene, spectinomycin resistance gene, kanamycin resistance gene, hygromycin resistance gene, and puromycin resistance gene.

It is preferred that the polynucleotide contains coding sequences such as a coding sequence for an insect olfactory receptor co-receptor, a coding sequence for a chromogenic or luminescent protein, and a coding sequence for a drug-resistant gene in the form of an expression cassette. The structure of the expression cassette is similar to that of the expression cassette for the sensor protein. The promoter of the expression cassette can be shared among multiple coding sequences.

The polynucleotide is preferably integrated into the genomic DNA (particularly preferably chromosomal genomic DNA). This enables stable expression of the sensor protein, making it suitable for chemical detection. In this case, the polynucleotide can be a single continuous region within the genomic DNA or a combination of two or more continuous regions (e.g., a form in which the sensor protein-coding sequence is included in continuous region A, and the coding sequence for a drug-resistant gene is included in continuous region B, which is separate from continuous region A, or a form in which the sensor protein-coding sequence is included in both continuous region A and continuous region B).

In another embodiment, the polynucleotide may be in a state in which it is not integrated into the genomic DNA. In this case, the polynucleotide can be in the form of a vector, for example. In this case, the polynucleotide can be a single polynucleotide molecule, or two or more polynucleotide molecules (e.g., a form in which the sensor protein-coding sequence is included in polynucleotide molecule A, while the coding sequence for a drug-resistant gene is included in polynucleotide molecule B, which is a separate molecule from polynucleotide molecule A, or a form in which the sensor protein-coding sequence is included in both polynucleotide molecule A and polynucleotide molecule B).

The cell is not particularly limited. From the perspective of chemical detection suitability, the cell is preferably animal cells such as insect cells or mammalian cells, with insect cells being particularly preferred.

The insect cells for use include, for example, Sf cells, MG1 cells, High Five^{™} cells, and BmN cells. Sf cells for use include, for example, Sf9 cells (ATCC CRL1711) and Sf21 cells. Insect cells, which possess coding sequences for insect odorant receptors in their genome, are usable as chemical sensors. Of the insect cells, those derived from insects of the family *Arctiidae* are particularly preferred.

The cells derived from insects of the family *Arctiidae* are not particularly limited as long as they are primary cultured cells or established cell lines of biological constituent cells derived from insects of the family *Arctiidae.*

Examples of the family *Arctiidae* include subfamilies such as *Arctiinae, Lithosiinae,* and *Syntominae,* among which the subfamily *Arctiinae* is preferred. The subfamily *Arctiinae* is, for example, preferably the genus *Spilosoma, Spilarctia,* or *Rhagonis,* with the genus *Spilosoma* being particularly preferable. While there is no particular limitation to the genus *Spilosoma, Spilosoma imparilis* is particularly preferable.

The cells derived from insects of the family *Arctiidae* can be obtained from known biological banks or collected and cultured from living insects of the family *Arctiidae* according to or with reference to known methods; if necessary, they can be established as cell lines.

Examples of cells derived from *Spilosoma imparilis* include FFPRI-SpIm-2AM-SF cells (MAFF No.: 275052) and FFPRI-SpIm-2AM-IPL411 cells (MAFF No.: 275053) from the Genebank Project, National Agriculture and Food Research Organization.

The method for culturing cells is not particularly limited, and a method according to or with reference to known methods for culturing cells can be used.

The culture medium used for culture may be either a natural medium or a synthetic medium as long as it contains carbon sources and inorganic salts that can be metabolized by the cells and is capable of efficiently culturing cells.

The carbon sources can be any substance that can be used by cells. Examples include carbohydrates, such as glucose, fructose, sucrose, molasses containing these sugars, starch, and starch hydrolysates; organic acids, such as acetic acid and propionic acid; and alcohols, such as ethanol and propanol.

The nitrogen sources for use can be, for example, inorganic or organic acid ammonium salts, such as ammonia, ammonium chloride, ammonium sulfate, ammonium acetate, and ammonium phosphate, other nitrogen-containing compounds, as well as peptone, meat extracts, yeast extracts, corn steep liquor, casein hydrolysates, soybean meal, soybean meal hydrolysates, various fermented microbial cells, or their digests.

The inorganic salts for use can be, for example, monopotassium phosphate, dipotassium phosphate, magnesium phosphate, magnesium sulfate, sodium chloride, iron(II) sulfate, manganese sulfate, copper sulfate, or calcium carbonate.

More specific examples of mediums, for example, for insect cells, include TNM-FH medium (manufactured by PharMingen), Sf-900 III SFM medium (manufactured by Life Technologies Corporation), ExCell400 and ExCell405 (both manufactured by JRH Biosciences), and Grace's Insect Medium (Nature, 195, 788 (1962)).

The pH of the medium is not particularly limited as long as it is within a range suitable for culturing cells. For example, for insect cells, the pH is preferably 6 to 7.

The culture temperature is not particularly limited as long as it is within a range suitable for culturing cells. For example, for insect cells, the culture temperature is preferably 25 to 30°C.

The culture vessel is not particularly limited as long as it is suitable for the production of cells for measuring the activity of a sensor protein. From the standpoint of suitability for the production of such cells, the preferred culture vessel is a cell culture flask (e.g., T25 flask, T75 flask, T175 flask, and T225 flask).

The amount of culture medium is not particularly limited; however, from the standpoint of suitability for the production of cells for measuring the activity of a sensor protein, the amount of culture medium can be preferably 0.16 to 0.24 mL, and more preferably 0.18 to 0.22 mL per square centimeter of the culture area. For example, in the case of a T75 flask (culture surface area: 75 cm²), the amount of culture medium can preferably be 12 to 18 mL, and more preferably 13.5 to 16.5 mL.

The material of the culture vessel is not particularly limited. For example, a material such as plastic (e.g., polystyrene) or glass can be used. The culture surface may be artificially treated, as necessary, to improve its adhesion with cells (e.g., coating treatment with extracellular matrices, such as basement membrane preparations, laminin, entactin, collagen, or gelatin, or with polymers, such as polylysine or polyornithine, or surface modification, such as positive-charge treatment), or may be artificially treated to reduce its adhesion with cells (e.g., super-hydrophilic treatment with an MPC polymer or low-protein-adsorption treatment).

The production method of the present disclosure is characterized by collecting the cells when the ratio of the activity value of the sensor protein at a concentration of 1 µM of a response substance to the activity value of the sensor protein at a concentration of 0 µM of the response substance (specific activity) is 1.2 or greater. Based on the finding that while the activity of the sensor protein may be almost completely lost due to culture, it is possible to increase the activity, the characteristic cell collection can be performed to obtain cells for measuring the activity of a sensor protein that can be used as a chemical sensor.

The activity value can be measured according to or with reference to the method described for Test Example 2 or Test Example 3, described below. In activity measurements in which the activity of the sensor protein is converted into light emitted from cells, "activity value" is synonymous with "light intensity value."

The response substance is a substance whose presence can be detected by the sensor protein. The response substance can be a ligand when the sensor protein is a receptor protein. The response substance for each sensor protein is either known or can be readily identified or determined based on known information.

When there are multiple response substances for a sensor protein, the substance with the highest specific activity can be selected.

For example, cells containing an exogenous polynucleotide that includes an expression cassette of a specific sensor protein (sensor protein X) are seeded and cultured to achieve a cell concentration of 0.89 × 10⁵ cells/mL. The cells are collected at a cell concentration of 9.0 × 10⁵ cells/mL. If the ratio of the activity value (specific activity) at a concentration of 1 µM of response substance x to the activity value at a concentration of 0 µM of response substance x is 1.2 or greater (preferably 1.3 or greater, more preferably 1.4 or greater, even more preferably 1.5 or greater, and yet more preferably 1.7 or greater) in these collected cells, response substance x can be determined to be a response substance for sensor protein X.

In an embodiment of the production method of the present disclosure, the cell concentration at the time of cell collection is preferably less than 2.9 × 10⁶ cells/mL. This facilitates the acquisition of cells with a specific activity at or above a predetermined level. From the viewpoint of specific activity, the cell concentration is preferably 2.2 × 10⁶ cells/mL or less, more preferably 2.0 × 10⁶ cells/mL or less, even more preferably 1.9 × 10⁶ cells/mL or less, still more preferably 1.8 × 10⁶ cells/mL or less, particularly preferably 1.75 × 10⁶ cells/mL or less, yet more preferably 1.7 × 10⁶ cells/mL or less, and especially preferably 1.65 × 10⁶ cells/mL or less. Of these concentrations, from the viewpoint of specific activity, the cell concentration is more preferably 1.2 × 10⁶ cells/mL or less, even more preferably 1.0 × 10⁶ cells/mL or less, and yet more preferably 9.5 × 10⁵ cells/mL or less. Furthermore, from the viewpoint of specific activity, the cell concentration is preferably 2.0 × 10⁵ cells/mL or more, more preferably 3.0 × 10⁵ cells/mL or more, even more preferably 4.0 × 10⁵ cells/mL or more, still more preferably 5.0 × 10⁵ cells/mL or more, yet more preferably 6.0 × 10⁵ cells/mL or more, particularly preferably 7.0 × 10⁵ cells/mL or more, even further more preferably 8.0 × 10⁵ cells/mL or more, and especially preferably 8.7 × 10⁵ cells/mL or more. Any range made by combining the above upper limits and lower limits can also be considered to be a suitable range.

In the present specification, the "cell concentration" refers to the total number of cells (the sum of live cells and countable dead cells of insect cells) per milliliter of culture medium.

In one embodiment, the present disclosure relates to a method for producing cells for measuring the activity of a sensor protein, comprising culturing cells containing a polynucleotide containing a coding sequence for the sensor protein, and collecting the cells when the cell concentration is less than 2.9 × 10⁶ cells/mL.

In an embodiment of the production method of the present disclosure, it is preferred that the culture period from the passage immediately before cell collection to the cell collection be 6 to 12 days. This facilitates the acquisition of cells with a specific activity at or above a predetermined level. The culture period is preferably 6 to 11 days, more preferably 7 to 11 days, and even more preferably 7 to 10 days.

In an embodiment of the production method of the present disclosure, the method preferably includes seeding the insect cells so as to achieve a cell concentration of 0.8 × 10⁵ to 2.0 × 10⁵ cells/mL at the passage immediately before the cells are collected. This facilitates the acquisition of cells with a specific activity at or above a predetermined level. From the viewpoint of specific activity, the cell concentration is preferably 0.8 × 10⁵ to 1.5 × 10⁵ cells/mL, more preferably 0.8 × 10⁵ to 1.4 × 10⁵ cells/mL, even more preferably 0.8 × 10⁵ to 1.3 × 10⁵ cells/mL, still more preferably 0.8 × 10⁵ to 1.2 × 10⁵ cells/mL, yet more preferably 0.8 × 10⁵ to 1.1×10⁵ cells/mL, particularly preferably 0.8 × 10⁵ to 1.0 × 10⁵ cells/mL, and especially preferably 0.8 × 10⁵ to 0.95 × 10⁵ cells/mL.

In an embodiment, the present disclosure relates to a method for producing cells for measuring the activity of a sensor protein, comprising culturing cells containing a polynucleotide containing a coding sequence of the sensor protein, and collecting the cells, and further comprising seeding insect cells so as to achieve a cell concentration of 0.8 × 10⁵ to 2.0 × 10⁵ cells/mL at the time of the passage immediately before the cell collection.

The obtained cell for activity measurement is preferably incorporated in a cell chip. Thus, in an embodiment, the present disclosure relates to a cell chip comprising one or more compartments each containing the cell for activity measurement ("the cell chip of the present disclosure" in the present specification).

The compartments of the cell chip of the present disclosure preferably contain cells and a hydrogel. This protects the cells from desiccation.

The configuration of the compartments is not particularly limited as long as it is capable of retaining the cells. From the perspectives of cell retention, production efficiency, or chemical detection, the compartments are preferably in the form of wells.

The material of the compartments is not particularly limited as long as the material can retain cells. The material can be, for example, resin or metal.

From the perspective of detection sensitivity, each compartment typically contains multiple cells. The number of cells per unit area (cm²) in a compartment is, for example, 1 × 10³ to 1 × 10⁹ cells/cm².

From the perspective of detection sensitivity or production efficiency, the bottom surface area of a single compartment is preferably 0.5 to 100 mm², more preferably 1 to 30 mm², and even more preferably 1.5 to 10 mm².

From the perspective of detection sensitivity or production efficiency, the number of compartments included in the cell chip is preferably 10 to 2000, more preferably 30 to 1000, and even more preferably 50 to 500.

It is preferred that the cell chip of the present disclosure includes two or more (more preferably three or more, even more preferably four or more, still more preferably five or more, ten or more, 15 or more, or 20 or more) types of cells that differ from each other in the type of sensor protein.

The cell for activity measurement and the cell chip of the present disclosure can be used in the detection of chemical substances (in particular, odorous substances). The chemical substances can be, for example, those in a sample such as body fluids (e.g., urine, blood, and saliva), air (e.g., indoor air and air inside packaging), and water (e.g., river water, seawater, tap water, clean water, and sewage). In this case, for example, the cell for activity measurement is brought into contact with the sample, or the sample is added to compartments of the cell chip of the present disclosure; this allows the chemical substance in the sample to reach the cells and come into contact with the sensor protein in the cells. For example, a chemical substance can be detected by detecting ions flowing into a cell (e.g., by detecting a protein that becomes colored or luminescent in response to ions).

### Examples

The present invention will be described in detail below with reference to Examples. However, the present invention is not limited to these Examples.

### Test Example 1: Preparation of Stably Expressing Cell

Cells derived from *Spilosoma imparilis* (SpIm cells) were transfected with a transposon vector containing the coding sequence for an odorant receptor protein (ORA), the coding sequence for an olfactory receptor co-receptor, the coding sequence for a calcium sensor fluorescence protein, and the coding sequence for a puromycin resistance gene that were all placed under control of a promoter sequence and that were positioned between the 5' ITR (inverted terminal repeat) and 3' ITR. Selection was performed with puromycin, thereby preparing SpIm cells stably expressing odorant receptors in which the foreign DNA containing the above coding sequences and promoter sequence was integrated into the chromosomal genomic DNA ("ORA cells" below). The odorant receptor is an insect-derived odorant receptor and is a receptor for compound a. The ORA cells emit fluorescence in response to compound a.

### Test Example 2: Production Test for Cells for Measuring Sensor Protein Activity 1

ORA cells were seeded to give a cell concentration of 0.8 × 10⁵ to 1.6 × 10⁵ cells/mL in culture medium (15 mL/flask, Sf-900III SFM medium) in a T75 flask (manufactured by Corning), and cultured for 7 to 16 days in a 27°C incubator (without CO₂ supply). After the medium used for culture was removed, 10 mL of fresh medium was added, followed by collecting the cells. The total number of cells at that time was measured using a fluidlab R-300 cell counter (anvajo GmbH).

The activity of the odorant receptor in the collected cells (ORA cells) was measured. Specifically, the details are as follows. ORA cells were seeded in a 96-well plate (Corning 3903) at 5 × 10⁴ cells/100 µL/well and left to stand in a 27°C incubator (without CO₂ supply). Sf-900III SFM medium was used for the culture medium. Cell counting was performed using a fluidlab R-300 cell counter (anvajo GmbH). After 24 hours from seeding, the medium was removed from the 96-well plate and replaced with 80 µL of 0.1% BSA/1×Hanks' buffer/20 mM HEPES buffer. After five minutes from the fluid exchange operation, the fluorescence intensity (GCaMP) was measured using a FlexStation 3 microplate reader. Specifically, compound a (a substance to which ORA cells respond) was added to each well to achieve a final concentration of 1 µM, and the changes in fluorescence intensity before and after the addition were quantified by using a microplate reader (FlexStation3, Molecular Devices). The ratio (specific activity) of the fluorescence intensity (activity value) in ORA cells after the addition of compound a (when the concentration of compound a was 1 µM) to the fluorescence intensity (activity value) before the addition of compound a (when the concentration of compound a was 0 µM) was calculated.

Table 1 shows the results.

**Table 1**

| Cell Concentration at the Time of Collection | Specific Activity |
|---|---|
| 3.6E+05 | 1.49 |
| 4.3E+05 | 1.40 |
| 5.0E+05 | 1.34 |
| 8.0E+05 | 1.54 |
| 8.5E+05 | 1.53 |
| 8.8E+05 | 1.58 |
| 9.0E+05 | 1.78 |
| 1.6E+06 | 1.42 |
| 1.9E+06 | 1.40 |
| 2.9E+06 | 1.00 |
| 3.2E+06 | 1.00 |

The results indicate that while the activity of sensor protein may be almost completely lost due to culture, it is possible to increase the activity. The results also indicate that the activity can be further enhanced by adjusting the cell concentration at the time of cell collection.

The activity was also measured with the concentration of compound a increased to 100 µM in the cases of cell concentrations of 2.9 × 10⁶ cells/mL and 3.2 × 10⁶ cells/mL at the time of collection; the specific activity was approximately 1.

### Test Example 3: Production Test for Cells for Measuring Sensor Protein Activity 2

ORA cells were seeded to give a cell concentration of 0.44 × 10⁵ cells/mL, 0.89 × 10⁵ cells/mL, 1.33 × 10⁶ cells/mL, or 1.78 × 10⁶ cells/mL in culture medium (4.5 mL/flask, Sf-900 III SFM medium) in a T25 flask (manufactured by Corning), and culture was initiated in a 27°C incubator (without CO₂ supply). After seven days from the start of culture, the culture medium was removed, and then 1.2 mL of fresh culture medium was added, followed by collecting the cells. The total number of cells at that time was measured using a fluidlab R-300 cell counter (anvajo GmbH).

The activity of the odorant receptor in the collected cells (ORA cells) was measured. Specifically, the details are as follows. ORA cells were seeded in a 96-well plate (Corning 3903) at 5 × 10⁴ cells/100 µL/well and left to stand in a 27°C incubator (without CO₂ supply). Sf-900III SFM medium was used for the culture medium. Cell counting was performed using a fluidlab R-300 cell counter (manufactured by anvajo GmbH). After 24 hours from seeding, the culture fluid was removed from the 96-well plate and replaced with 80 µL of 0.1% BSA/1×Hanks' buffer/20 mM HEPES buffer. After five minutes from the culture fluid exchange operation, the fluorescence intensity (GCaMP) was measured using a FlexStation 3 microplate reader. Specifically, compound a (a substance to which ORA cells respond) was added to each well to achieve a final concentration of 1 µM, and the changes in fluorescence intensity before and after the addition were quantified by using a microplate reader (FlexStation3, Molecular Devices). The ratio (specific activity) of the fluorescence intensity (activity value) in ORA cells after the addition of compound a (when the concentration of compound a was 1 µM) to the fluorescence intensity (activity value) before the addition of compound a (when the concentration of compound a was 0 µM) was calculated.

The results revealed that the specific activity was greater when the cell concentration was 0.89 × 10⁵ cells/mL, 1.33 × 10⁶ cells/mL, and 1.78 × 10⁶ cells/mL as compared to when the cell concentration was 0.44 × 10⁵ cells/mL at the time of seeding. In particular, when the cell concentration was 0.89 × 10⁵ cells/mL or 1.33 × 10⁶ cells/mL, the specific activity was notably high, with the specific activity being 1.62 at a cell concentration of 0.89 × 10⁵ cells/mL and 1.43 at a cell concentration of 1.33 × 10⁶ cells/mL, as compared to when the cell concentration was 1.78 × 10⁶ cells/mL.

## Claims

1. A method for producing a cell for measuring activity of a sensor protein, comprising
culturing a cell containing a polynucleotide containing a coding sequence for the sensor protein, and
collecting the cell when the ratio of the activity value of the sensor protein at a concentration of 1 µM of a response substance to the activity value of the sensor protein at a concentration of 0 µM of the response substance (specific activity) is 1.2 or greater.

2. The method according to claim 1, wherein the cell concentration at the time of the cell collection is less than 2.9 × 10⁶ cells/mL.

3. The method according to claim 2, wherein the cell concentration at the time of the cell collection is 2.2 × 10⁶ cells/mL or less.

4. The method according to claim 3, wherein the cell concentration at the time of the cell collection is 2.0 × 10⁵ cells/mL or more.

5. The method according to claim 4, wherein the cell concentration at the time of the cell collection is 8.7 × 10⁵ to 1.75 × 10⁶ cells/mL.

6. The method according to claim 1, wherein the culture period from a passage immediately before the cell collection to the cell collection is 6 to 12 days.

7. The method according to claim 1, comprising seeding the insect cell so as to achieve a cell concentration of 0.8 × 10⁵ to 2.0 × 10⁵ cells/mL at the time of a passage immediately before the cell collection.

8. The method according to claim 7, wherein the cell concentration is 0.8 × 10⁵ to 1.5 × 10⁵ cells/mL.

9. The method according to any one of claims 1 to 8, wherein the cell is an insect cell.

10. The method according to any one of claims 1 to 8, wherein the sensor protein is an odorant receptor protein.

11. A cell comprising a polynucleotide containing a coding sequence for a sensor protein,
wherein the cell is obtained through a culture step,
and
wherein the ratio of the activity value of the sensor protein at a concentration of 1 µM of a response substance to the activity value of the sensor protein at a concentration of 0 µM of the response substance (specific activity) is 1.2 or greater.
